# NEUE EUROPÄISCHE PATENTSCHRIFT

(11) **EP 0 271 806 B2**
(45) Veröffentlichungstag und Bekanntmachung des Hinweises auf die Entscheidung über den Einspruch: **27.03.1996**
(45) Hinweis auf die Patenterteilung: 20.01.1993
(21) Anmeldenummer: 87118142.6
(22) Anmeldetag: 08.12.1987
(51) Int. Cl.: A61K 51/10

(54) **Verfahren zur Herstellung einer mit Technetium-99m-markierten organspezifischen Substanz**
Method for the preparation of a technetium-99m-labelled organ-specific substance
Méthode de préparation d'une substance spécifique d'organe marquée au technetium-99m

(30) Priorität: 10.12.1986 DE 3642173; 27.08.1987 DE 3728599
(43) Veröffentlichungstag der Anmeldung: 22.06.1988
(73) Patentinhaber: HOECHST AKTIENGESELLSCHAFT, D-65926 Frankfurt am Main (DE)
(72) Erfinder: Bremer, Karl-Heinz, Dr., D-6232 Bad Soden am Taunus (DE); Kuhlmann, Ludwig, Dr., D-6093 Flörsheim am Main (DE); Schwarz, Alexander, Dr., D-6093 Flörsheim am Main (DE); Steinsträsser, Axel, Dr. Dr., D-6237 Liederbach (DE)

(56) Entgegenhaltungen:
- EP-A- 0 005 638
- EP-A- 0 028 092
- EP-A- 0 035 265
- EP-A- 0 108 253
- EP-A- 0 111 414
- EP-A- 0 179 481
- EP-A- 0 194 853
- WO-A-85/03063
- FR-A- 2 281 134
- TUMOR IMAGING, 1982, Seiten 111-123, Masson Publishing, New York, US; B.A. RHODES et al.: "99mTc-labeling and acceptance testing of radiolabeled antibodies and antibody fragments"
- CHEMICAL ABSTRACTS, Band 103, 1985, Seite 342, Zusammenfassung Nr. 118993z, Columbus, Ohio, US; T.J.F. SAVELKOUL et al.: "Protein-binding and urinary excretion of technetium-99m(tin)-MDP and technetium-99m-MDP", & INT. J. NUCL. MED. BIOL. 1985, 12(2), 125-31
- CHEMICAL ABSTRACTS, Band 87, 1977, Seite 170, Zusammenfassung Nr. 35143x, Columbus, Ohio, US; M.K. DEWANJEE et al.: "Correlation of protein binding and the localization of technetium-99m-labeled pyrophosphate and other agents in infarcted myocardium", & J. LABELLED COMPD. RADIOPHARM. 1977, 13(2), 251
- APPLIED RADIATION AND ISOTOPES, Band 31, Nr. 8, August 1980, Seiten 499-504, Pergamon Press Ltd, Oxford, GB; M.W. BILLINGHURST et al.: "Determination of the optimal concentrations of stannous pyrophosphate for in vivo red blood cell labelling with technetium-99m"
- J.Nuclear Med. 1975, vol. 16, p. 435-437
- J.Nucl. Med. 1974, vol. 17, p. 380-384
- Cancer Research, vol. 40 (1980) p. 3043-3045

## Beschreibung

Gegenstand der Erfindung ist ein Verfahren zur Herstellung einer mit Technetium-99m-markierten organspezifischen Substanz, ein zu ihrer Herstellung geeigneter Testkit und ein die markierte organspezifische Substanz enthaltendes Diagnostikum.

Nach den bahnbrechenden Arbeiten von Milstein und Köhler, die 1975 ein Verfahren publizierten, bei dem Antikörper sezernierende Maus-B-Lymphozyten mit Maus-Myelomzellen verschmolzen werden und das Fusionsprodukt beider Zellen, das Hybrid, weiterwächst und Antikörper erzeugt, ist es möglich, aus der Vielzahl entstehender Hybride dasjenige zu isolieren, welches einen Antikörper bestimmter Spezifität produziert. Diese monoklonalen Antikörper, die nur ein bestimmtes Epitop auf dem entsprechenden Antigen erkennen, stehen heute in größerer Menge für die Immunszintigraphie zur Verfügung. Monoklonale Antikörper gegen tumorassoziierte Antigene eignen sich für die Tumorlokalisationsdiagnostik sowie für den Nachweis von Rezidiven und Metastasen. Hierzu muß der Antikörper vor der Injektion radioaktiv markiert werden, um mit geeigneten nuklearmedizinischen Aufnahmetechniken festzustellen, ob sich ein das Antigen tragender Tumor im Organismus befindet. Von den heute in der nuklearmedizinischen Diagnostik am häufigsten eingesetzten Radionukliden sind Jodisotope zur Markierung von Proteinen am einfachsten einsetzbar. Dabei erfüllt Jod-123 am ehesten die in der Praxis gestellten Forderungen, wie gute Nachweisempfindlichkeit und geringe Strahlenbelastung, da es ein reiner γ-Strahler ist und eine günstige γ-Energie von 159 keV besitzt. Seine relativ kurze Halbwertszeit von 13,2 Stunden und vor allem seine an Teilchenbeschleuniger gebundene Herstellung, durch die auch ein relativ hoher Preis bedingt ist, schränken den Einsatz des Jod-123 zur Antikörpermarkierung stark ein.

Mit Jod-131, das wegen einer für Gammakameras energetisch ungünstigen γ-Strahlung und seiner aus der Sicht der Strahlenbelastung her unerwünschten β-Strahlung kein ideales Radionuklid für die in vivo-Diagnostik ist, werden vielfach Antikörpermarkierungen durchgeführt, weil J-131 in hoher Aktivitätskonzentration mit ausreichend hoher spezifischer Aktivität kostengünstig zu erhalten und dadurch eine relativ einfache und preiswerte Markierung zu erreichen ist.

Der entscheidende Nachteil aller jodmarkierten Antikörper besteht darin, daß das radioaktive Jod in vivo teilweise durch Dejodierungsprozesse wieder aus dem Antikörper abgespalten wird und im Organismus dann als Jodid vorliegt, das einerseits in der Schilddrüse gespeichert wird und andererseits die Untergrundaktivität erhöht, da Jodid relativ langsam aus dem Blut eliminiert wird. Bei der Applikation jodmarkierter Antikörper muß in jedem Fall die Schilddrüse der zu untersuchenden Person vorher blockiert werden. Ein gewisser Vorteil jodmarkierter Antikörper zeigt sich darin, daß ihre Speicherung in Leber und Nieren zum üblichen Untersuchungszeitpunkt deutlich niedriger ist als beim gleichen, jedoch mit anderen Radionukliden markierten Antikörper.

In-111 wird neuerdings vielfach zu Antikörpermarkierungen herangezogen. Als metallisches Element läßt sich Indium im Gegensatz zu Jod nicht ohne weiteres an Proteine binden. Man benötigt hierzu bifunktionelle Chelatbildner, die einerseits eine kovalente Bindung mit dem Protein eingehen und andererseits über eine starke, komplexbildende Gruppe das als Kation vorliegende Indium an den Antikörper binden können. Das am häufigsten hierzu benutzte Komplexon ist Diethylentriaminpentaessigsäure (DTPA). DTPA wird als bicyclisches Anhydrid mit dem Antikörper umgesetzt. Dabei geht es zunächst eine kovalente Amidbindung mit endständigen Aminogruppen des Proteins ein, während anschließend durch Reaktion mit Wasser seine restliche Säurefunktion freigesetzt wird. Der so derivatisierte Antikörper kann nun das Radionuklid, das als In-111-citrat zugesetzt wird, fest binden. Das Indium muß als labile Komplexverbindung angeboten werden, da es sonst bei den erforderlichen pH-Werten ausfallen würde. Das bicyclische Anhydrid des DTPA ist kein ideales Reagenz, da es über seine Bifunktionalität inter- und intramolekulare Kopplungsreaktionen eingehen kann.

Während Gallium im Vergleich zu Indium keine Vorteile aufweist, wird die Technetium-99m-Markierung von potentiellen Diagnostika angestrebt, da Tc-99m wegen seiner günstigen physikalischen Eigenschaften (Fehlen einer Korpuskularstrahlung, γ-Energie von 140 keV und Halbwertszeit von 6 Stunden) und der damit verbundenen geringen Strahlenbelastung zum wichtigsten Radionuklid in der nuklearmedizinischen Diagnostik geworden ist.

Technetium-99m, das sich aus Nuklidgeneratoren gewinnen läßt, liegt zunächst als Pertechnetat vor, das in dieser Form, z.B. für die Schilddrüsen- und Hirnszintigraphie geeignet ist. Die Szintigraphie anderer Organe mittels Technetium-99m gelingt mit Hilfe bestimmter "Transportsubstanzen", die einerseits in der Lage sind, Technetium zu binden, und andererseits das Radionuklid im Zielorgan mit hoher Selektivität anzureichern. Zu diesem Zweck werden bisher vorwiegend Substanzen eingesetzt, die direkt mit Technetium-99m markierbar sind und eine hohe Organspezifität aufweisen. Darüber hinaus gibt es jedoch eine Reihe von Substanzen, die zwar eine hohe Organspezifität aufweisen, aber nicht direkt markierbar sind. Dies können Proteine (Fibrinogen, Humanserumalbumin), Enzyme (Streptokinase, Lactatdehydrogenase), Zucker (Dextran, Glucose) oder auch Polymere sein. Dazu gehören ebenfalls niedermolekulare Substanzen, wie Fettsäuren, die durch den hohen Energiebedarf des Herzens sich im Myocardgewebe anreichern.

Zur Markierung der organspezifischen "Transportsubstanz" mit Technetium-99m muß das aus dem Nuklidgenerator eluierte Pertechnetat zuerst in eine niedrigere Oxidationsstufe übergeführt werden. In dieser reduzierten Form bildet Technetium mit der organspezifischen Substanz mehr oder weniger stabile Verbindungen. Für die Knochenszintigraphie werden z.B. Tc-99m-Phosphorsäure-Derivate, vor allem organische Phosphonsäuren eingesetzt. So liegt in der im Europäischen Patent 2485 beschriebenen Markierungseinheit das Natriumsalz der 3,3-Diphosphono-1,2-propandicarbonsäure als organspezifische "Transportsubstanz" vor. In dem europäischen Patent 108.253 werden Tc-99m-Tri- und Tetraphosphonsäuren zur szintigraphischen Darstellung des RES, insbesondere der Leber, beschrieben. Der Tc-99m-Komplex mit Diethylentriaminpentaessigsäure (DTPA) findet zur Diagnostik von Nierenerkrankungen bzw. pathologischen Hirnprozessen Anwendung.

Da eine direkte Markierung von z.B. Antikörpern mit Technetium nicht möglich ist, hat man versucht, ähnlich wie bei der Indium-Markierung, unter Zuhilfenahme von bifunktionellen Komplexbildnern, eine stabile Technetiummarkierung der Antikörper zu erreichen. Das besondere Problem bei der Markierung mit Techetium besteht darin, daß normalerweise Zinn-II-Ionen in der Reaktionslösung vorhanden sind. Zinn-II ist bisher das einzige Reduktionsmittel, das eine schnelle und quantitative Überführung des Pertechnetats bei Raumtemperatur in eine niedrigere und dadurch reaktionsfähige Oxidationsstufe ermöglicht. Die neben dem reduzierten Technetium vorliegenden Zinn-II- und -IV-Ionen konkurrieren um die Bindungsstellen des an den Antikörper gekoppelten Komplexes, so daß entweder der Komplexbildner im Überschuß eingesetzt werden muß, wodurch die spezifischen Eigenschaften des Antikörpers beeinflußt werden können, oder ungebundenes Technetium und Zinn als gemeinsames Kolloid zu unerwünschter Radioaktivitätsspeicherung in anderen Organen führt.

In dem US-Patent 4.479.930 werden die cyclischen Anhydride vom DTPA und EDTA als Chelatisierungsmittel nicht nur für In-111 und Ga-67, sondern auch für Tc-99m angeführt. In dem europäischen Patent 35765 wird die Verwendung von Deferoxamin als Komplexierungsagens für Technetium-99m an Proteine erwähnt. In der internationalen Patentanmeldung WO 85/3063 werden die partiell reduzierten Disulfid-Brücken im Antikörper mit dem Natriumsalz des Tetrachlornitridotechnetats, das durch Reaktion von Pertechnetat mit Natriumazid vorher präpariert werden muß, umgesetzt.

Einfachere Methoden zur Tc-99m-Markierung von Antikörpern bzw. Antikörperfragmenten sind in dem europäischen Patent 5638 und dem US-Patent 4.478.815 beschrieben. Dort werden Zinn-II-salze im Überschuß zur gleichzeitigen reduktiven Spaltung von Disulfid-Brücken und zur Reduktion des zugesetzten Tc-99m-Pertechnetats benutzt. Im allgemeinen benötigt man zur Spaltung der -S-S-Bindung längere Inkubationszeiten (24 h), wobei F(ab')₂-Fragmente partiell zu F(ab')-Fragmenten gespalten werden. Neuere Literaturangaben (z.B. Journal of Nuclear Medicine 27 (1986), Seite 685 - 93 und 1315 - 20 sowie International Journal of Nuclear Medicine Biology 12 (1985) Seiten 3 - 8) zeigen, daß das Verhältnis der beiden Fragmente abhängig ist von der "Bezinnungsreaktion und daß sich das Verhältnis der beiden Komponenten nach der Tc-99m-Markierung nicht mehr nennenswert ändert, wobei die Hauptkomponente Tc-99m-markiertes F(ab') ist. In allen Fällen mußte das markierte F(ab')-Fragment nachgereinigt werden, da trotz mindestens 30-minütiger Reaktionszeit keine quantitative Umsetzung des Pertechnetats erreicht wurde.

Die EP-A- 0 0179 481 beschreibt die Verwendung von Sn²⁺-Ionen zur In-vivo-Tc-99m-Markierung von Erythrozyten und von Sn²⁺-Kolloid, markiert mit Tc-99m, zur Leberszintigraphie. Um SnCl₂-Lösungen vor Hydrolyse und Oxidation zu schützen, werden konzentrierte SnCl₂-Lösungen in sehr kleine Volumina abgefüllt und lyophilisiert. Bei der Markierung sind Sn²⁺-Ionen durch die Ausbildung eines Tc-Sn-Kolloids zwingend beteiligt.

Nach den oben beschriebenen Markierungsverfahren war es bisher nicht möglich, Präparate herzustellen, die routinemäßig ohne aufwendige Verfahrensschritte eingesetzt werden können.

Es wurde nun ein Verfahren zur Herstellung einer mit Technetium-99m markierten organspezifischen Substanz gefunden, bei dem ein vorbehandelter oder ein mit einem komplexbildner für Technetium-99m gekoppelter Antikörper oder ein F(ab')₂-Antikörperfragment als organspezifische Substanz, mit Pertechnetat-99m und einem Zinn-II-Phosphonat oder -Pyrophosphat als komplexstabilisiertes Reduktionsmittel versetzt wird.

Auf diese Weise können organspezifischen Substanzen ("Trägersubstanzen") m it Technetium-99m markiert werden.

Die zu markierende Verbindung muß - vor der Markierung - "vorbehandelt" oder an einen geeigneten Komplexbildner gekoppelt werden.

Unter "vorbehandelt" werden im Sinne der Erfindung solche Maßnahmen verstanden, die dazu führen, daß eine funktionelle Gruppe mit komplexbildenden Eigenschaften im zu markierenden Molekül entstehen. Beispielsweise enthalten Antikörper Disulfid-Brücken. Die beiden kovalent miteinander verknüpften Schwefelatome sind jedoch in dieser Form nicht befähigt, Technetium-99m zu komplexieren. Reduziert man jedoch die Disulfid-Brücke, so entstehen zwei SH-Gruppen, die nun ihrerseits ausgzeichnete Komplexliganden für Technetium-99m darstellen und diese auch in guten Ausbeuten binden.

Eine andere Möglichkeit Technetium-99m an organspezifische Substanzen, die keine funktionelle Gruppe mit komplexierenden Eigenschaften besitzt, zu binden, besteht darin, eine solche funktionelle Gruppe in das Molekül einzubauen oder ein Komplexierungsagenz an das Molekül chemisch zu binden.

Ein wesentlicher Punkt der Erfindung liegt darin, daß die zu markierende Substanz nicht direkt mit dem Zinn-II-Salz in Kontakt kommt. Zunächst wird das Zinn-II-Salz getrennt davon mit einem geeigneten Reaktionspartner versetzt, wobei ein komplexstabilisiertes Reduktionsmittel gebildet wird. Der Reaktionspartner ist ein Phosphonat oder eine Pyrophosphat, das Zinn-II bei einem physiologischen pH-Wert (6-8) in Lösung hält und somit das Reduktionsmittel in funktionsfähigem Zustand erhält (komplexstabilisiertes Reduktionsmittel).

Das Verfahren findet für die Technetium-99m-Markierung von Antikörpern Anwendung. Eine partielle Reduktion der S-S-Bindungen des Antikörpers oder eines F(ab')₂-Antikörperfragments läßt sich bei Raumtemperatur durch kurzzeitige Einwirkung von milden Reduktionsmitteln erreichen (Vorbehandeln der organspezifischen Substanz). Besonders geeignete Reduktionsmittel sind Monothiole wie 2-Mercaptoethanol oder 2-Mercaptoethylamin (Cysteamin). Dabei erhält man reaktionsfähige Antikörpermoleküle, die weder ihre immunologische Reaktivität eingebüßt haben noch zu kleineren Bruchstücken fragmentiert worden sind. Grundsätzlich sind für die partielle Reduktion des Antikörpers oder des F(ab')₂-Antikörperfragments alle Reduktionsmittel geeignet, die auch bei längerer Einwirkungszeit nur einen Teil der S-S-Bindungen spalten und zu keiner Fragmentierung der Antikörperkomponente führen. Die Einwirkungszeit eines derartigen Reduktionsmittels auf die Antikörperkomponente braucht eine Stunde nicht zu übersteigen. Im allgemeinen sind schon nach 10 bis 30 Minuten so viele SH-Gruppen entstanden, daß ausreichende Mengen von Technetium-99m-Kationen gebunden werden. Dann wird das überschüssige Reduktionsmittel abgetrennt und der partiell reduzierte Antikörper in einer gepufferten Lösung (z.B. 0,02 M Phosphatlösung, pH 7,2) vereinzelt und umgehend lyophilisiert. Dabei muß eine Reoxidation der freien Thiolgruppen im Antikörper durch Luft-Sauerstoff unterbunden werden. Der lyophilisierte Antikörper, der außer den Puffersalzen keine weiteren Zusätze enthält und mit Stickstoff als Schutzgas überlagert wird, ist bei Kühlschranktemperatur (-5 bis +5°C) wochenlang unverändert haltbar; er löst sich bei Zugabe von isotonischer Natriumchloridlösung einwandfrei wieder auf.

Die so hergestellte, partiell reduzierte Antikörperkomponente (vorbehandelte organspezifische Substanz) kann nun glatt mit Technetium-99m markiert werden, wenn man ihr ein Gemisch aus Pertechnetat- und Zinn-II-phosphonat oder -pyrophosphat zusetzt. Besonders vorteilhaft ist es, wenn man als Zinn-II-phosphonate, Diphosphonate, Triphosphonate oder Tetraphosphonate einsetzt. Ganz besonders bewährt haben sich die Zinn-II-Salze der Methandiphosphonsäure, der Aminomethandiphosphonsäure, der 3,3-Diphosphonopropionsäure, der 3,3-Diphosphono-1,2-propandicarbonsäure oder der Propan-1,1,3,3-tetraphosphonsäure. Diese zinnhaltigen Phosphonatkomplexe sind bereits in den europäischen Patenten 24 85 und 108 253 beschrieben und finden als Technetium-Markierungsbesteck für die Skelett- und Leberszintigraphie breite Anwendung. Ebenso geeignet ist Zinn-II-haltiges Pyrophosphat, während das Zinn-II-Salz der Diethylentriaminpentaessigsäure ungeeignet ist.

Zur Herstellung eines einsatzbereiten Diagnostikums kann man nun so vorgehen, daß man zuerst die lyophilisierte Antikörperkomponente in einer Technetium-99m-Pertechnetatlösung auflöst und dann die Reduktion und Bindung des Technetiums an den Antikörper durch Zugabe einer Lösung der Zinn-II-Komponente bewirkt.

Man kann das Diagnostikum aber auch dadurch herstellen, daß man die Antikörperkomponente zuerst in der Zinn-II-haltigen Lösung auflöst und anschließend durch Zugabe von Technetium-99m-Pertechnetatlösung den Antikörper mit Technetium markiert.

Zur Herstellung eines Diagnostikums, welches eine Technetium-99m-markierte organspezifische Substanz enthält, wird zweckmäßigerweise ein Testkit zusammengestellt, der zwei getrennte, vorzugsweise lyophilisierte Komponenten enthält, von denen eine die organspezifische Substanz bzw. die vorbehandelte organspezifische Substanz oder die mit einem Komplexbildner für Tc-99m gekoppelte, organspezifische Substanz, ggf. im Gemisch mit einem Puffer und die andere das komplexstabilisierte Zinn-II-Salz enthält, das zur Reduktion und Bindung des Technetiums an der organspezifischen Substanz benötigt wird. Besonders bewährt hat sich ein Testkit, bei dem die lyophilisierte, gegebenenfalls vorbehandelte organspezifische Substanz im Gemisch mit Dinatriumhydrogenphosphat (pH 7,2) als Puffersubstanz vorliegt. Man erhält so nach kurzer Reaktionszeit, z.B. schon nach 5 Minuten, eine nahezu quantitative Technetium-99m-Markierung der Substanz, die weniger als 1 % freies Pertechnetat und nur sehr geringe Mengen der Tc-99m-markierten Zinn-II-Komponente als Verunreinigungen enthält, so daß anschließende Reinigungsverfahren nicht mehr erforderlich sind.

Die getrennte Abfüllung der organspezifischen Substanz und der Zinn-II-Komponente gewährleistet eine unveränderte Haltbarkeit des lyophilisierten Präparats. Damit ist eine schnelle, unproblematische und einwandfreie Markierung der organspezifischen Substanz gewährleistet. Die dafür zur Reduktion des Pertechnetats benötigte Menge an Zinn-II ist naturgemäß gering, in der Größenordnung von einigen Mikrogramm. Vom zinnhaltigen Phosphonat oder Pyrophosphat werden, bezogen auf Zinn-II, jeweils 1 bis 100 Mikrogramm, vorzugsweise 5 bis 10 Mikrogramm pro 1 mg der organspezifischen Komponente zugesetzt, um eine stabile Markierung mit Technetium-99m zu erreichen.

Die erfindungsgemäß einzusetzenden Zinn-II-phosphonate oder -pyrophosphate sind für die Markierung von reduzierten Antikörpern oder reduzierten Antikörperfragmenten besonders gut geeignet, weil sie bei dem für die Markierung erforderlichen neutralen pH-Wert einen stabilen Komplex bilden, der jedoch das reduzierte Technetium-Kation nur locker bindet, so daß es leicht mit den Thiolgruppen im Antikörper oder dessen Fragment ausgetauscht werden kann.

Der in manchen Markierungsbestecken vorhandene Stabilisator ist auch bei der Antikörpermarkierung von Vorteil, da er eine längere Haltbarkeit der Injektionslösung garantiert.

Durch die in der europäischen Patentanmeldung 141 100 beschriebene N-(4-Aminobenzoyl)-glutaminsäure, die in den für die Skelettszintigraphie eingesetzten Diphosphonaten als stabilisierende Komponente verwendet werden kann, läßt sich eine ausgezeichnete Haltbarkeit der mit Technetium-99m-markierten Antikörper erreichen.

Der erfindungsgemäß hergestellte Technetium-99m-markierte Antikörper oder sein F(ab')₂-Antikörperfragment finden bevorzugt Anwendung zum in-vivo-Nachweis von Tumoren. Vorzugsweise verwendet man einen monoklonalen Antikörper oder dessen F(ab')₂-Fragmente, die mit Tumor-assoziierten Antigenen reagieren.

### Beispiel 1

20 mg des gegen das carcino-embryonale Antigen (CEA) gerichteten monoklonalen Antikörpers BW 431/31, der zum diagnostischen Nachweis von kolorektalen Karzinomen Verwendung findet, wurden bei Raumtemperatur durch Dialyse von Zusätzen wie Sucrose befreit und in isotonische Natriumchloridlösung übergeführt. Dieser Antikörper ist in der deutschen Offenlegungsschrift 34 16 774 und von Bosslet et al., Int. J. of Cancer 36, 75 - 84 (1985) beschrieben.

Der anschließend in einer Konzentration von ca. 5 mg pro ml vorliegende Antikörper wurde mit insgesamt 20 mg 2-Mercaptoethanol bei Raumtemperatur (30 Minuten) umgesetzt und danach durch Gelfiltration an Bio-Gel P-2, einem Polyacrylamid-Gel der Fa. Bio Rad, vom überschüssigen Reduktionsmittel getrennt. Der in 0,02 M Phosphatpuffer (pH 7,2) gelöste Antikörper wurde umgehend vereinzelt und lyophilisiert. Die gefriergetrockneten Proben enthielten pro Abfüllung 2 mg des Antikörpers und etwa 1,4 mg Dinatriumhydrogenphosphat.

Zur Technetium-99m-Markierung wurden die Proben jeweils mit 7,5 ml Pertechnetatlösung mit insgesamt bis zu 3000 MBq (etwa 80 mCi) umgesetzt. Als Zinn-II-Komponente wurde eine lyophilisierte Markierungseinheit bestehend aus 5 mg des Natriumsalzes der 3,3-Diphosphonopropionsäure, 0,1 mg Zinn-II-Kationen und 0,5 mg N-(4-Aminobenzoyl)-L-glutaminsäure, eingesetzt. Dieses Gemisch wurde in 5 ml physiologischer Natriumchloridlösung gelöst und direkt anschließend (nach einer Minute) 0,5 ml dieser Lösung zu der Antikörperlösung zugefügt, so daß das Gesamtvolumen 8 ml betrug. Nach 10-minütiger Reaktionszeit wurde die Markierungsausbeute durch Dünnschichtchromatographie, Gelfiltration auf Biogel P-10 und Hochdruck-Flüssigkeitschromatographie an Zorbax GF 250 der Fa. DuPont geprüft.

Über 95 % der Technetium-99m-Aktivität war proteingebunden, ca. 1 % lag als phosphonatgebundene Anteile vor, während weniger als 1 % Pertechnetat waren. Der Pertechnetatanteil stieg erst bei längerer Standzeit wieder an; nach 6 Stunden lag er bei rund 2 %, während der phosphonatgebundene Anteil unverändert bei 1 % lag.

Die Bestimmung des immunreaktiven Anteils in verschiedenen Technetium-99m-markierten Antikörpern durch Messung der Bindung an Tumorzellen ergab gut übereinstimmende Werte für die Immunreaktivität mit entsprechenden mit Jod-131 und Indium-111 markierten CEA-Antikörpern (Tabelle 1).

**Tabelle 1**

| Vergleich der Immunreaktivität (%) von monoklonalen Antikörpern, die mit den Radionukliden J-131, In-111 und Tc-99m markiert wurden. | | | |
|---|---|---|---|
| Monoklonaler Antikörper | J-131 | In-11 | Tc-99m |
| BW 431/31 | 85 | 75 | 90 |
| BW 431/26 | 85 | 70 | 95 |
| Bw 494/32 | 65 | 45 | 70 |

Der in der Tabelle genannte monoklonale Antikörper Bw 431/26 ist aus der DE-A-34 16 774 bekannt und wird dort als MAK VIII bezeichnet. Der monoklonale Antikörper BW 494/32 ist in der DE-A- 35 31 301 beschrieben.

### Beispiel 2 (Vergleichsbeispiel)

Der monoklonale Antikörper BW 431/31 wurde in derselben Weise wie in Beispiel 1 behandelt, jedoch nicht mit 2-Mercaptoethanol umgesetzt. Der deshalb keine freien Thiolgruppen enthaltende Antikörper ergab dann bei Reaktion mit Pertechnetat in Gegenwart einer gleichen Menge Zinndiphosphonat, durchgeführt wie in Beispiel 1, ein Produkt, bei dem nur ca. 1 % des Technetium-99m- proteingebunden war, während der größere Anteil (mehr als 50 %) als Technetium-99m-diphosphonat vorlag und neben reduziertem, ungebundenem Technetium (31 %) noch ein erheblicher Anteil an freiem Pertechnetat (15 %) vorhanden war.

### Beispiel 3

Zur Technetium-99m-Markierung des gegen ein Pankreaskarzinom-assoziiertes Mucusantigen gerichteten monoklonalen Antikörpers BW 494/32 wurden 20 mg dieser Substanz, gelöst in 2 ml isotonischer Natriumchloridlösung, mit 0,5 ml einer 1 %igen wäßrigen Cysteaminhydrochloridlösung 20 Minuten bei Raumtemperatur inkubiert. Der säulenchromatographisch gereinigte, modifizierte Antikörper, gelöst in 0,02 M
Na₂HPO₄-Pufferlösung, wurde in Portionen zu je 2 mg lyophilisiert. Als Zinn-II-Komponente zur Technetiummarkierung des Antikörpers wurde ein Pyrophosphat-Markierungsbesteck, das 7,2 mg Na₄P₂O₇ und 1,03 mg Zinn-II-chlorid pro Kit enthält, benutzt. Der Inhalt einer Abfüllung wurde in 10 ml physiologischer Natriumchloridlösung gelöst und 0,25 ml, entsprechend etwa 14 µg Sn²⁺, dieser Lösung dem Antikörper, der vorher in etwa 8 ml Pertechnetatlösung (etwa 2000 MBq) gelöst worden war, zugesetzt. Nach 10-minütiger Reaktionszeit wurden jeweils 0,1 ml der Lösung mit folgender Zusammensetzung

| | |
|---|---|
| 25 µg | mab |
| 2,25 µg | Na₄P₂O₇ |
| 0,175 µg | Zinn-II |
| und ca. 18 µg | Na₂HPO₄-Puffer |

gesunden Ratten iv. appliziert. Die Organverteilung 24 Stunden post injectionem (p.i) ist in Tabelle 2 gezeigt. Daraus geht hervor, daß die Speicherungen in Knochen, Schilddrüse und Magen sehr gering und in den übrigen Organen dem mit Jod-131-markierten Antikörper vergleichbar sind. Für die Immunreaktivität (vgl. Tabelle 1) wurde beim Technetium-99m-markierten Antikörper BW 494/32 ein höherer Wert als bei Markierung mit Jod-131 oder Indium-111 gemessen.

**Tabelle 2**

| Organverteilung von Tc- 99m- und J-131-mab 494/32 in normalen Wistarratten 24 h p.i. (in % der applizierten Dosis pro Organ bzw. pro Gramm Gewebe). | | |
|---|---|---|
| Organ | Tc-99m | J-131 |
| Leber %/g | 0,49 | 0,23 |
| Lunge | 0,82 | 0,48 |
| Milz | 0,65 | 0,30 |
| Nieren | 4,25 | 0,70 |
| Knochen | 0,57 | 0,20 |
| Blut | 1,97 | 0,92 |
| Muskel | 0,12 | 0,08 |
| Magen % gesamt | 0,38 | 2,50 |
| Schilddrüse | 0,05 | 4,85 |
| Darm | 3,83 | 4,62 |
| Urin | 24,5 | 49,3 |

### Beispiel 4

Vom ebenfalls gegen CEA gerichteten monoklonalen Antikörper BW 431/26 wurden 50 mg durch Behandlung mit 2-Mercaptoethanol partiell reduziert, gereinigt und in 2 mg-Abfüllungen in Gegenwart von Phosphatpuffer (pH 7,2) lyophilisiert.

Die Tc-99m-Markierung wurde mit Hilfe einer sonst für die Leberszintigraphie benutzten Markierungseinheit, bestehend aus 13,5 mg 1,1,3,3,-Propantetraphosphonsäuretetranatriumsalz und 0,6 mg Zinn-II-chlorid x 2H₂O, durchgeführt. Von dem in 10 ml isotonischer NaCl-Lösung aufgelösten Inhalt einer Markierungseinheit, wurden 0,5 ml, entsprechend 15 µg Sn²⁺, zu dem lyophilisierten Antikörper gegeben. Anschließend wurde Pertechnetat-Lösung (3000 MBq) zu der klaren Antikörper-Sn-II-salz-lösung zugesetzt und der Tc-99m-markierte Antikörper Nacktmäusen mit einem implantierten humanen Kolonkarzinom iv. appliziert. Szintigraphisch war der Tumor bereits 24 Stunden p.i. gut darstellbar. Tabelle 3 zeigt anhand der Tumorspeicherung und Organvertielung dieses Antikörpers in der Nacktmaus, daß die mit J-131, In-111 und Tc-99m-markierten Präparate vergleichbar sind.

**Tabelle 3**

| Tumorspeicherung und Organverteilung von I-131-, In-111 und Tc-99m-mab 431/26 in Nacktmäusen (n = 2) mit humanem Kolonkarzinom in Abhängigkeit von der Zeit | | | | | | |
|---|---|---|---|---|---|---|
| post injectionem | J-131 | | In-111 | | Tc-99m | |
| (p.i.) (h) | 17 | 48 | 17 | 48 | 17 | 30 |
| Tumor %/g | 14,9 | 8,8 | 9,8 | 13,0 | 11,0 | 14,9 |
| Leber %/g | 4,6 | 3,0 | 8,5 | 8,4 | 7,2 | 5,4 |
| Nieren %/g | 3,4 | 1,9 | 12,2 | 16,4 | 10,6 | 7,7 |
| Muskel %/g | 1,3 | 0,84 | 1,4 | 1,2 | 0,9 | 1,0 |
| Blut %/ml | 14,6 | 13,0 | 20,5 | 9,2 | 17,0 | 15,0 |
| Knochen % gesamt | 1,8 | 1,7 | 4,7 | 4,3 | 2,4 | 2,1 |

### Beispiel 5

20 mg des F(ab')₂-Fragments vom monoklonalen Antikörper 431/31 wurden bei 4° C 15 Minuten mit 1 ml einer 1 %igen Cysteaminhydrochloridlösung inkubiert. Das vom überschüssigen Reduktionsmittel befreite Antikörperfragment wurde zusammen mit Phosphatpuffer lyophilisiert. Zur Markierung mit Technetium wurde eine Markierungseinheit, bestehend aus 13,0 mg 3,3-Diphosphono-1,2-propandicarbonsäuretetranatriumsalz, 0,23 mg Zinn-II-oxid und 1,0 mg N-(4-Aminobenzoyl)-L-glutaminsäuremononatriumsalz, von der 1/20 in 0,5 ml physiologischer Kochsalzlösung verwendet wurde, eingesetzt. Das mit einer spezifischen Aktivität von 1000 MBq/mg markierte Antikörperfragment enthielt praktisch kein Pertechnetat, jedoch einen geringfügig höheren (ca. 3 %) Anteil an Tc-99m-Diphosphonat. Die Immunreaktivität lag bei 60 %.

## Patentansprüche

1. Verfahren zur Herstellung einer mit Technetium-99m markierten organspezifischen Substanz, dadurch gekennzeichnet, daß ein vorbehandelter oder ein mit einem Komplexbildner für Technetium-99m gekoppelter Antikörper oder ein F(ab')₂-Antikörperfragment als organspezifische Substanz mit Pertechnetat-99m und einem Zinn-II-Phosphonat oder -Pyrophosphat als komplexstabilisiertes Reduktionsmittel versetzt wird.

2. Verfahren nach Anspruch 1, dadurch gekennzeichnet, daß als Zinn-II-phosphonate Diphosphonate, Triphosphonate oder Tetraphosphonate eingesetzt werden.

3. Verfahren nach Anspruch 2, dadurch gekennzeichnet, daß Zinn-II-Salze der Methandiphosphonsäure, Aminomethandiphosphonsäure, 3,3-Diphosphonopropionsäure, 3,3-Diphosphono-1,2-propandicarbonsäure oder der Propan-1,1,3,3-tetraphosphonsäure eingesetzt werden.

4. Verfahren nach Anspruch 1, dadurch gekennzeichnet, daß man einen monoklonalen Antikörper oder dessen F(ab')₂-Fragment einsetzt.

5. Verfahren nach Anspruch 3, dadurch gekennzeichnet, daß man einen Antikörper oder dessen F(ab')₂-Fragment gegen Tumor assoziierte Antigene einsetzt.

6. Verfahren zur Herstellung eines Testkits, dadurch gekennzeichnet, daß man zwei getrennte, lyophilisierte Komponenten, Von denen eine eine organspezifische Substanz gemäß Anspruch 1 und die andere das Reduktionsmittel enthält, das zur Reduktion und Bindung des Technetiums an die organspezifische Substanz benötigt wird, in eine geeignete Gebrauchsform bringt.

7. Verfahren zur Herstellung eines Testkits nach Anspruch 6, dadurch gekennzeichnet, daß die organspezifische Substanz ein partiell reduzierter Antikörper oder dessen F(ab')₂-Fragment ist.

8. Verfahren zur Herstellung eines Testkits nach Anspruch 7, dadurch gekennzeichnet, daß die lyophilisierte Antikörperkomponente im Gemisch mit Dinatriumhydrogenphosphat als Puffersubstanz Vorliegt.

9. Verfahren zur Herstellung eines Diagnostikums, dadurch gekennzeichnet, daß man zuerst die Komponente, die die organspezifische Substanz gemäß Anspruch 1 enthält, in einer Technetium-99m-Pertechnetat-Lösung auflöst und dann die Reduktion und Bindung des Technetiums an die organspezifische Substanz durch Zugabe Von Zinn-II-Phosphonat oder -Pyrophosphat als komplexstabilisiertes Reduktionsmittel bewirkt.

10. Verfahren zur Herstellung eines Diagnostikums, dadurch gekennzeichnet, daß man die Komponente, die die organspezifische Substanz gemäß Anspruch 1 enthält, zuerst in der Lösung Von Zinn-II-Phosphonat oder -Pyrophosphat auflöst und anschließend durch Zugabe Von Technetium-99m-Pertechnetat-Lösung die organspezifische Substanz mit Technetium markiert.

11. Verfahren zur Herstellung eines Diagnostikums nach Anspruch 9 oder 10, dadurch gekennzeichnet, daß von dem komplexstabilisierten Reduktionsmittel, bezogen auf Zinn-II, jeweils 1 bis 100 Mikrogramm, Vorzugsweise 5 bis 10 Mikrogramm pro 1 mg der organspezifischen Substanz verwendet werden, um diese stabil mit Technetium-99m zu markieren.

## Claims

1. A process for the preparation of an organ-specific substance labeled with technetium-99m, which comprises mixing an antibody or a F(ab')₂ antibody fragment, as organ-specific substance, which has been pretreated or coupled to a complexing agent for technetium-99m, with pertechnetate-99m and a tin-II phosphonate or tin-II pyrophosphate as complex-stabilized reducing agent.

2. The process as claimed in claim 1, wherein diphosphonates, triphosphonates or tetraphosphonates are used as tin-II phosphonates.

3. The process as claimed in claim 2, wherein tin-II salts of methanediphosphonic acid, aminomethanediphosphonic acid, 3,3-diphosphonopropionic acid, 3,3-diphosphono-1,2-propanedicarboxylic acid or propane-1,1,3,3-tetraphosphonic acid are used.

4. The process as claimed in claim 1, wherein a monoclonal antibody or its F(ab')₂ fragment is used.

5. The process as claimed in claim 3, wherein an antibody, or its F(ab')₂ fragment, against tumor-associated antigens is used.

6. A process for the production of a test kit, which comprises converting two separate, freeze-dried components, one of which contains an organ-specific substance as claimed in claim 1 and the other contains the reducing agent which is required for the reduction and binding of the technetium to the organ-specific substance, into a form suitable for use.

7. The process for the production of a test kit as claimed in claim 6, wherein the organ-specific substance is a partially reduced antibody or its F(ab')₂ fragment.

8. The process for the production of a test kit as claimed in claim 7, wherein the freeze-dried antibody component is mixed with disodium hydrogen phosphate as buffer substance.

9. A process for the production of a diagnostic aid, which comprises initially dissolving the component which contains the organ-specific substance as claimed in claim 1 in a technetium-99m-pertechnetate solution, and then bringing about the reduction and binding of the technetium to the organ-specific substance by addition of tin-II phosphonate or tin-II pyrophosphate as complex-stabilized reducing agent.

10. A process for the production of a diagnostic aid, which comprises initially dissolving the component which contains the organ-specific substance as claimed in claim 1 in the solution of tin-II phosphonate or tin-II pyrophosphate, and then labeling the organ-specific substance with technetium by addition of technetium-99m-pertechnetate solution.

11. The process for the production of a diagnostic aid as claimed in claim 9 or 10, wherein in each case 1 to 100 micrograms, preferably 5 to 10 micrograms, based on tin-II, of the complex-stabilized reducing agent are used for each 1 mg of the organ-specific substance in order for the labeling of the latter with technetium-99m to be stable.

## Revendications

1. Procédé de préparation d'une substance spécifique d'organe marquée au technétium-99m, caractérisé en ce ou'un anticorps prétraité ou couplé à un agent complexant pour le technétium-99m ou bien un fragment d'anticorps F(ab')₂ en tant que substance spécifique d'organe est mélangé à du pertechnétate-99m et un phosphonate ou un pyrophosphate stanneux en tant que réducteur stabilisé sous forme complexe.

2. Procédé selon la revendication 1, caractérisé en ce que les phosphonates stanneux utilisés sont des diphosphonates, des triphosphonates ou des tétraphosphonates.

3. Procédé selon la revendication 2, caractérisé en ce que l'on utilise des sels stanneux de l'acide méthanediphosphonique, de l'acide méthylaminediphosphonique, de l'acide 3,3-diphosphonopropionique, de l'acide 3,3-diphosphono-1,2-propanedicarboxylique ou de l'acide propane-1,1,3,3-tétraphosphonique.

4. Procédé selon la revendication 1, caractérisé en ce que l'on utilise un anticorps monoclonal ou le fragment F(ab')2 de celui-ci.

5. Procédé selon la revendication 3, caractérisé en ce crue l'on utilise un anticorps dirigé contre des antigènes associés à une tumeur ou le fragment F(ab')₂ de celui-ci.

6. Procédé de préparation d'un nécessaire d'analyse, caractérisé en ce que l'on met sous forme appropriée à l'utilisation deux composants lyophilisés séparés dont l'un contient une substance spécifique d'organe selon la revendication 1 et l'autre contient le réducteur nécessaire à la réduction et à la fixation du technétium sur la substance spécifique d'organe.

7. Procédé de préparation d'un nécessaire d'analyse selon la revendication 6, caractérisé en ce que la substance spécifique d'organe est un anticorps partiellement réduit ou le fragment F(ab')2 de celui-ci.

8. Procédé de préparation d'un nécessaire d'analyse selon la revendication 7, caractérisé en ce que le composant d'anticorps lyophilisé se présente en mélange avec du phosphate disodique utilisé comme tampon.

9. Procédé de préparation d'un agent de diagnostic, caractérisé en ce crue l'on dissout d'abord le composant contenant la substance spécifique d'organe selon la revendication 1 dans une solution de pertechnétate de technétium-99m, puis l'on effectue la réduction et la fixation du technétium sur la substance spécifique d'organe en ajoutant du phosphonate ou du pyrophosphate stanneux en tant que réducteur stabilisé sous forme complexe.

10. Procédé de préparation d'un agent de diagnostic, caractérisé en ce que le composant contenant la substance spécifique d'organe selon la revendication 1 est d'abord dissous dans la solution de phosphonate ou de pyrophosphate stanneux, et la substance spécifique d'organe est ensuite marquée au technétium en ajoutant une solution de pertechnétate de technétium-99m.

11. Procédé de préparation d'un agent de diagnostic selon la revendication 9 ou la revendication 10, caractérisé en ce que l'on utilise 1 à 100 microgrammes, de préférence 5 à 10 microgrammes, de réducteur stabilisé sous forme complexe, ramené à l'étain II, pour 1 mg de substance spécifique d'organe afin de marquer celle-ci au technétium-99m de manière stable.
